Europäisches Patentamt

European Patent Office

Office européen des brevets

(19)

(11) Publication number: **0 087 161**
**A2**

## EUROPEAN PATENT APPLICATION

(12)

(21) Application number: 83101683.7

(22) Date of filing: 22.02.83

(51) Int. Cl.³: **A 61 K 31/23**
**A 61 K 31/05**
**//(A61K31/23, 31/05)**

(30) Priority: 24.02.82 US 351627
17.05.82 US 378807

(43) Date of publication of application:
31.08.83 Bulletin 83/35

(84) Designated Contracting States:
AT BE CH DE FR GB IT LI LU NL SE

(71) Applicant: Key Pharmaceuticals, Inc.
18425 N.W. 2nd Avenue
Miami, Florida 33169(US)

(72) Inventor: Keith, Alec D.
18425 N.W. 2nd Avenue
Miami Florida 33169(US)

(74) Representative: Werner, Hans-Karsten, Dr. et al,
Deichmannhaus am Hauptbahnhof
D-5000 Köln 1(DE)

(54) Isopropylmyristate 4-alkyl-2,6-(bis-tert-butyl)-phenol medicament.

(57) Herpes infections are treated with a mixture comprising a major amount of isopropylmyristate and a minor amount of a 4-(lower alkyl)-2,6-(bis-*tert*-butyl)-phenol, with 2,6-(bis-*tert*-butyl)-4-hydroxy-toluene being a preferred embodiment of the 4-(lower alkyl)-2,6-(bis-*tert*-butyl)-phenol.

EP 0 087 161 A2

## ISOPROPYLMYRISTATE 4-ALKYL-2,6-(BIS-TERT-BUTYL)-PHENOL MEDICAMENT

### Background of the Invention

4-(Lower alkyl)-2,6-(bis-tert-butyl)-phenols are known anti-herpes agents, the compound 2,6-(bis-tert-butyl)-4-hydroxy-toluene having been described several years ago in Cupp et al., "Inactivation of the Lipid-Containing Bacteriophage PM2 by Butylated Hydroxytoluene", Antimicrobial Agents and Chemotherapy, Vol. 8, no. 6, pp. 698-706 (1975), with subsequent higher homologs in this series, e.g., 2,6-(bis-tert-butyl)-4-butylphenol, being in the prior art for the same activity.

### Summary of the Invention

The present invention comprises a major portion by weight of isopropylmyristate and a minor portion by weight of 4-(lower alkyl)-2,6-(bis-tert-butyl)-phenol, which provides an anti-herpes effect when substituted for the formulation of Keith et al., U.S. Patent No. 4,350,707, granted September 21, 1982 (Serial No. 655,413, filed February 5, 1976). As the 4-lower alkyl substituent may be mentioned methyl, ethyl, propyl, butyl and amyl as representative members of the grouping.

In a preferred embodiment there is provided from about 5% to about 30%, still more preferably about 10% to about 15% of 4-(lower alkyl)-2,6-(bis-_tert_-butyl)-phenol by weight and from about 70% to about 95%, and still more preferably about 85% to about 90% by weight of isopropylmyristate. In this embodiment, a typical application would be to apply about 60 microliters per day to the area where blisters, sores or lesions occur, over four applications per day.

In a further aspect of the present invention, and as a prophylactic against trauma caused by sun that is particularly important for those that may only occasionally receive large and sudden amounts of ultraviolet radiation (e.g. weekend ski vacationers), a composition is provided which includes from about 10% to about 30% 4-(lower alkyl)-2,6-(bis-_tert_-butyl)-phenol and about 5% to about 20% isopropylmyristate in a "lipstick" formulation that is based upon a pharmaceutically compatible lipspreadable vehicle and contains a minor amount of sunscreen agent. This embodiment of the invention provides both a normal protection for the lips of a person exposed to extreme cold and also protection against a recurrent herpes simplex infection. As the pharmaceutically compatible lip-spreadable solid vehicle may be mentioned various compatible polymers, with polyethylene glycol having a molecular weight of from about 5,000 to about 30,000 being mentioned as one example, with a molecular weight of about 10,000 being preferred. The minor amount of the sunscreen that is contemplated in this aspect of the present invention is up to about 5% of the sunscreen, with about 2% being preferred in accordance with one embodiment. As the sunscreen may be mentioned conventionally used sunscreens of sun lotion formulations. In accordance with a preferred embodiment, it is contemplated that as the sunscreen there may be used 4-hydroxycinnamic acid or dioctyl-para-aminobenzoic acid.

## Detailed Description of the Invention

Herpes simplex has become a disease of increasing importance in an era of sexual freedom and an apparent security felt by the public until quite recently that venereal diseases, even though formerly quite serious and without cures, are now readily disposed of through an injection of penicillin or other antibiotic. Treatment of both herpes I and herpes II are contemplated in this invention. While penicillin and other drugs have proven extremely effective in the treatment of the traditionally important venereal diseases, such treatments are not effective against the herpes virus. The herpes virus attacks the nerve cell nucleus, generally in the spinal ganglia, with no way having been found to date to attack this infestation of the cell nucleus by the invading herpes virus. When a trauma occurs, the herpes virus travels from the cell nucleus through the nerve trunk where it had originally penetrated, generally at the lips of the mouth or the genitalia, providing an unwelcome and often quite painful blister, sore or lesion. Patients who have suffered a trauma, whether physical (a high fever or ultraviolet radiation) or psychological (a shock due to business or personal problems) may anticipate the outbreak of a herpes attack. The trauma that may trigger this recurrent manifestation of the disease, and the period when the victim is most able to transmit his disease to one in intimate contact, is at the time of the outbreak. It is thus not only bothersome to the victim to have to undergo such an outbreak of this disease, but he furthermore is more prone to pass the disease on to an intimate partner, either through heavy kissing or close touching of the genitals, and not even necessarily through sexual intercourse. The blisters, sores or lesions are generally limited to the areas of contact. Spreading of the disease from one area to another is observed, for example, in children who may touch an infected area and transfer the infection to another. Dentists frequently contract herpes in their fingers, due to work in the mouths of patients having herpes infections. Transfer of herpes between the oral and genital regions has increased with the apparent increase in sexual contacts between these regions. While less frequent in terms of absolute numbers, but much more serious from a medical standpoint, is the situation where herpes infects the newborn and in some cases results in a terminal infection (such herpes being contracted through passage of the baby through an infected vaginal tract as part of delivery). Herpes of the eye, where blindness may result, is also serious. The following examples serve to illustrate the invention:

## EXAMPLE I

A topical anti-herpes agent is prepared by adding to isopropylmyristate sufficient 2,6-(bis-tert-butyl)-4-hydroxy-toluene to provide a mixture which by weight constitutes 15% 2,6-(bis-tert-butyl)-4-hydroxy-toluene and 85% isopropylmyristate. Animal tests corresponding to those conducted in the Keith et al. patent indicate improved penetration of the sites of herpes infections versus the Keith et al. patent where the topical agent contains 5% 2,6-(bis-tert-butyl)-4-hydroxy-toluene, 50% ethanol, 37% propylene glycol, 3% water and Tween 20.

The same total amount of the composition of the present invention may be substituted for the total amount of the composition of the Keith et al. patent to provide an effective anti-herpes topical agent.

## EXAMPLE II

Animal tests were conducted with hairless mice applying the 85% isopropylmyristate - 15% 2,6-(bis-tert-butyl)-4-hydroxy-toluene mixture of Example I topically to the sites where lesions would be expected to develop or actually were present. A beneficial effect in the respective avoidance and clearing up of lesions was observed. The amount of the total composition to be applied to the area of an actual or anticipated lesion outbreak in a human is 15 microliters, once every six hours. Application is particularly indicated as a prophylactic against the outbreak of lesions, this dosage to be particularly applied during the occurrence of a fever, exposure to ultraviolet light, or emotional trauma, when outbreaks are likely to occur.

## EXAMPLE III

Substituting an equal amount of 4-ethyl-2,6-(bis-tert-butyl)-phenol for the 2,6-(bis-tert-butyl)-4-hydroxy-toluene of Example I, an antiherpes topical medicament is obtained, which may be used in accordance with the procedure of Example II.

## EXAMPLE IV

Substituting an equal amount of 4-butyl-2,6-(bis-tert-butyl)-phenol for the 2,6-(bis-tert-butyl)-4-hydroxy-toluene of Example I, an antiherpes topical medicament is obtained, which may be used in accordance with the procedure of Example II.

## EXAMPLE V

A "lipstick"-type of medicine particularly suited for protection of persons exposed to large amounts of ultraviolet light is provided. In a base of 10,000 mw polyethylene glycol there are admixed by weight 10% isopropylmyristate, 15% 2,6-(bis-*tert*-butyl)-4-hydroxy-toluene and 2% 4-hydroxycinnamic acid. After mixing the ingredients thoroughly together, a solid having the consistency of a lipstick is formed into a lipstick shape for application to the lips of a patient about to receive the potential trauma of ultraviolet radiation, such as when on a ski vacation.

It is to be understood that this embodiment is designed for infected areas that are exposed to this particular trauma source, and, for example, this embodiment would not be contemplated for the genitalia.

WHAT IS CLAIMED IS:

1. A composition suitable for topical application to the site of lesion formation in a patient suffering from herpes simplex infection which comprises a major amount of isopropylmyristate and a minor amount of 4-(lower alkyl)-2,6-(bis-<u>tert</u>-butyl)-phenol.

2. A composition of claim 1, wherein said 4-(lower alkyl)-2,6-(bis-<u>tert</u>-butyl)-phenol is 2,6-(bis-<u>tert</u>-butyl)-4-hydroxy-toluene.

3. A composition of claim 1, wherein said 4-(lower alkyl)-2,6-(bis-<u>tert</u>-butyl)-phenol is 4-ethyl-2,6-(bis-<u>tert</u>-butyl)-phenol.

4. A composition of claim 1, wherein said 4-(lower alkyl)-2,6-(bis-<u>tert</u>-butyl)-phenol is 4-propyl-2,6-(bis-<u>tert</u>-butyl)-phenol.

5. A composition of claim 1, wherein said 4-(lower alkyl)-2,6-(bis-<u>tert</u>-butyl)-phenol is 4-butyl-2,6-(bis-<u>tert</u>-butyl)-phenol.

6. A composition of claim 1, wherein said 4-(lower alkyl)-2,6-(bis-<u>tert</u>-butyl)-phenol is present in an amount of from about 10 to about 20% by weight of the total composition.

7.  A composition of claim 1, 2 or 3, wherein said isopropylmyristate is present in an amount of about 85% by weight of the total composition.

8. A method of preventing the appearance of blisters, sores or lesions caused by the herpes simplex virus in a patient infected with said herpes simplex virus, said patient not manifesting the symptoms thereof, which comprises topically applying to said patient at a region where blisters, sores or lesions have previously appeared an effective amount of a mixture comprising a major portion of isopropylmyristate and a minor portion of 4-(lower alkyl)-2,6-(bis-tert-butyl)-phenol.

9. A method of claim 8, wherein said 4-(lower alkyl)-2,6-(bis-tert-butyl)-phenol is 2,6-(bis-tert-butyl)-4-hydroxy-toluene.

10. A method of claim 8, wherein said 4-(lower alkyl)-2,6-(bis-tert-butyl)-phenol is 4-ethyl-2,6-(bis-tert-butyl)-phenol.

11. A method of claim 8, wherein said 4-(lower alkyl)-2,6-(bis-tert-butyl)-phenol is 4-propyl-2,6-(bis-tert-butyl)-phenol.

12. A method of claim 8, wherein said 4-(lower alkyl)-2,6-(bis-tert-butyl)-phenol is 4-butyl-2,6-(bis-tert-butyl)-phenol.

13. A method of claim 8, wherein said 4-(lower alkyl)-2,6-(bis-tert-butyl)-phenol is present in an amount of from about 10 to about 20% by weight of the total composition.

14.  A method of claim 8, 9 or 13 wherein said isopropylmyristate is present in an amount of about 85% by weight of the total composition.

15. A composition suitable for avoidance of lip blisters in an orally-infected herpes victim to be exposed to ultraviolet radiation which comprises:

    (a) from about 10 to about 30% 4-(lower alkyl)-2,6-(bis-<u>tert</u>-butyl)-phenol;

    (b) from about 5 to about 20% isopropylmyristate;

    (c) up to about 5% of a sunscreen; and

    (d) a pharmaceutically compatible lip-spreadable solid vehicle.

16. A composition of claim 15, wherein said 4-(lower alkyl)-2,6-(bis-<u>tert</u>-butyl)-phenol is 2,6-(bis-<u>tert</u>-butyl)-4-hydroxy-toluene.

17. A composition of claim 15, wherein said 4-(lower alkyl)-2,6-(bis-<u>tert</u>-butyl)-phenol is 4-ethyl-2,6-(bis-<u>tert</u>-butyl)-phenol.

18. A composition of claim 15, wherein said 4-(lower alkyl)-2,6-(bis-<u>tert</u>-butyl)-phenol is 4-propyl-2,6-(bis-<u>tert</u>-butyl)-phenol.

19. A composition of claim 15, wherein said 4-(lower alkyl)-2,6-(bis-<u>tert</u>-butyl)-phenol is 4-butyl-2,6-(bis-<u>tert</u>-butyl)-phenol.

20. A composition of claim 16, wherein said 2,6-(bis-<u>tert</u>-butyl)-4-hydroxy-toluene is present in an amount of about 15%.

21. A composition of claim 15, wherein isopropylmyristate is present in an amount of about 15%.

22. A composition of claim 15, wherein said sunscreen is present in an amount of about 1%.

23. A composition of claim 22, wherein sunscreen is 4-hydroxycinnanic acid.

24. A composition of claim 15, wherein said lip-spreadable solid vehicle is polyethylene glycol having a molecular weight of from about 5,000 to about 30,000.